# EUROPEAN PATENT APPLICATION

(11) **EP 2 433 656 A1**
(43) Date of publication of application: **28.03.2012**
(21) Application number: 10178674.7
(22) Date of filing: 23.09.2010
(51) Int. Cl.: A61L 9/12, A61L 9/14

(54) **Fragrance dispensing device**

(71) Applicant: Unilever PLC, London, Greater London EC4Y 0DY (GB); Unilever NV, 3013 AL Rotterdam (NL)
(72) Inventor: Blanking, Pär Robert Erik William, Leeds, LS10 1PT (GB); Diamant, Benjamin Nathan, Leeds, LS14 2AR (GB); Thompson, Guy Richard, Leeds, LS14 2AR (GB)
(74) Representative: Whaley, Christopher

(57) **Abstract**

The invention relates to a hand-portable fragrance dispensing device (100) comprising a base (101) and a cartridge (102) configured for mechanically engagement with each other, where the cartridge is configured to contain a fragrance medium and the device comprises an electrically-driven dispenser (503) configured to dispense an aerosol of the fragrance medium from the cartridge under control from a driver (601) contained in the base, wherein the device comprises a communications interface (107) configured to communicate with a computer and is configured to transmit an identification code for the device over the communications interface when connected to the computer.

## Description

The invention relates to a hand-portable fragrance dispensing device comprising base and cartridge portions configured for mechanically engagement with each other, in which a fragrance medium contained in the cartridge is emitted from the device under control of an electrically-operated actuator.

Hand-portable fragrance dispensing devices are available in many different forms, common forms including a container having a manually operated atomiser or an unpressurised container with a pump-action aerosol dispenser. The container may be pressurised with a propellant, so that a fragrance medium contained in the device is emitted under pressure, creating a fine airborne spray, or aerosol, containing the fragrance together with the propellant and a carrier. The carrier may be water or alcohol-based and, if pressurised, may also contain a more volatile propellant such as a liquefied gas, for example propane, n-butane or isobutane.

A problem with existing pressurised fragrance dispensers relates to the use of a propellant, which results in atmospheric pollution and potential health hazards depending on the type of propellant. Alternative ways of creating an aerosol spray have been developed in recent years. One particular way involves the use of a vibrating membrane driven by a piezoelectric actuator, which allows for an aerosol spray to be generated directly from a liquid fragrance medium without the need for a propellant. An example of this type of fragrance dispenser is described in FR2929861 and FR2929862, each of which disclose a cartridge having a perforated vibrating membrane that is driven by a piezoelectric transducer for spraying a liquid contained in a reservoir.

An example of a fragrance dispensing device, or scent dispenser, in a hand-portable format is disclosed in US 2009/0054116, in which a scent dispenser comprising a piezoelectrically-driven vibrating perforated membrane is incorporated into a mobile handset. The use of piezoelectrically-driven membranes for dispensing fragrance addresses the problems associated with the use of propellants, while maintaining a fine droplet size that is not generally achievable with the use of finger-operated unpressurised dispensers.

A further example of a fragrance dispensing device in a hand-portable format is described in WO 2007/137483, which discloses a fragrance-emitting apparatus for use with a USB port, in which a heating element is operable to cause a fragrance to be emitted from a fragrance member. The device does not, however, have the advantages of a conventional scent dispenser, in that an aerosol is not generated by the device. Such devices, which rely on power from the USB connection to power a heating element are not therefore generally portable and are generally only able to dispense fragrance within a limited volume.

When using interchangeable cartridges in a fragrance dispensing device, it is important that a degree of uniformity in appearance is maintained between the part of the device that remains consistent and the part that is interchangeable, i.e. the cartridge. This may be achieved by having the cartridge held within a master unit, and therefore hidden when in use, but in some cases the cartridge may be more visible. It is preferable, however, for different cartridges, distinguished for example by containing different fragrances, to be readily visibly distinguishable from each other when in use, so that a user can easily tell which fragrance is contained in the cartridge. When mass-producing such cartridges, it would be preferable however to minimise on the number of changes in tooling that are required when changing from manufacture of one type of cartridge to another.

A further problem is how to determine a level of fragrance present in a cartridge and, related to this, how to inform a user that a fragrance medium in a cartridge is about to run out. This is a particular issue with electrically-activated devices, because of their generally smaller size and the use of additional components that may obscure the view of a liquid level within the cartridge. As an example, visible indications such as transparent windows that allow observation of a level of liquid inside the cartridge are not always possible due to design constraints. Windows may also add to the manufacturing cost of a design due to the need to incorporate additional components or an extra processing step. It would therefore be advantageous to be able to provide an indication to a user of the current state of a cartridge in use, preferably in an easily read format. In addition, when such level indicators are available, a user may have difficulty in seeing how much fragrance medium is left. An indication of the type of fragrance being used would also be advantageous.

A further problem is how to provide to a user of a fragrance dispensing device suggestions for further fragrances based on their personal preferences or use habits. Existing methods, such as carrying out surveys of users or focus groups, are time consuming and not necessarily accurate for individual users. A general lack of understanding among users of ways in which to describe fragrances also makes such surveys difficult to carry out and provide meaningful answers. Surveys are also often ignored, and the results may not be representative of an actual range of users.

A further problem related to use of electronically-actuated fragrance dispensing devices having interchangeable cartridges is that of how to operate a device having part of its functionality in a master, or base, portion and another part of its functionality in an interchangeable cartridge. Typically, for manufacturing cost reasons, the dispensing actuator will form part of the cartridge unit, while the driving electronics will form part of the master unit. The dispensing actuator on the cartridge may need to be protected when not in use, for example to prevent damage to the actuator by rough handling or from ingress of dust and dirt. An actuator cover is therefore preferable, but this results in the user being required to carry out at least two actions to cause the device to dispense, one action involving opening or unsealing the dispenser and another action causing the dispenser to operate. This may also be the case with non-electrically actuated dispensers, for example where an aerosol can is fitted with a locking device to prevent accidental use during transport. These additional actions can impair the attractiveness and usability of the device, as well as increase the complexity of the design.

It is an object of the invention to address one or more of the above mentioned problems.

According to a first aspect of the invention there is provided a hand-portable fragrance dispensing device comprising a base and a cartridge configured for mechanical engagement with each other, the cartridge configured to contain a fragrance medium, the device comprising an electrically-driven dispenser configured to dispense an aerosol of the fragrance medium from the cartridge under control from an electronic driver contained in the base,
wherein the device comprises a communications interface configured to communicate with a computer and is configured to transmit an identification code over the communications interface when connected to the computer.

The identification code may be unique to the cartridge and/or the base, so as to indicate to the computer the configuration and identity of the device being connected. The identification code may alternatively indicate to the computer merely the type of device, together with the type of cartridge, being connected, and allow a user to associate a unique identity with the device being connected. In either case, an advantage of this aspect of the invention is that a connection can be set up between a particular device and a computer that can be used for various purposes, including keeping track of how the device is used and updating the device with information via the communications interface that may be dependent on how the device is used.

The device may comprise a memory and be configured to log data on operation of the dispenser and store the data in the memory.

Data logged by the device and stored in the memory may for example relate to when, and for how long, the dispenser is operated, and what type of cartridge is being used. This data can be used to build up a profile of a user based on their use of the device. Data may also be logged on where the device is operated, if the device is configured to sense its geographical location, for example by means of a GNSS (Global Navigation Satellite Systems) receiver such as may commonly be incorporated into current hand-portable radio telephones.

An identification code may be provided on the base, the cartridge or on both portions of the device. An advantage of providing such identification codes is that identification of the base can be used to identify a user of the device and identification of the cartridge can be used to identify the type of fragrance being used. A combination of these codes can then be used to generate personalised data on the user, i.e. data on how the user is using the device.

The computer with which the device is configured to communicate may be a general purpose computer such as an x86-based personal computer, although other types of computing devices may also be used, examples being personal communication devices such as cell phones or personal digital assistants (PDAs), and including notebook or laptop-type computers and video game consoles. In general, the computer is a device that is able to communicate over the communications link available on the device and that is capable of executing software that allows user interaction with the device and preferably with a remote server. The computer is therefore preferably one that is connected, or is connectable, to the internet.

An advantage of the device having an identification code, being preferably unique for that device, which is transmitted to a computer when connected via the communications interface, is that a remote server can track usage of devices that have been connected in this way and use this information to provide feedback for the user of each device. This feedback may, for example, be in the form of marketing information such as recommendations for other cartridges that may be available or may be used to enable automated delivery of replacement cartridges for that user, in response to their personal and consumption specifications. The identification code may also be used to allow a user to gain access to a personalised internet site, through which a user's details may be available.

The device may comprise a memory and be configured to log data on usage of the device and to store the data in the memory. Having a memory in the device that is able to log usage of the device is advantageous because this can be used to develop more detailed profiles of a particular user of the device, for example in terms of when and how often the device is used and which fragrances are used. These details may also be transmittable over the communications interface and used by a remote server. This also has the advantage that the information can be stored temporarily on the device and then communicated to the computer/server on connection.

Optionally, the base comprises the memory, although in alternative embodiments the memory, or a part of the memory, may form part of the cartridge.

The communications interface may comprise a connection for a wired link to the computer. Exemplary wired links include USB (Universal Serial Bus) connections. In alternative embodiments, the communications interface is a wireless interface. Exemplary wireless interfaces include radiofrequency or infrared interfaces. Particular examples of wireless interfaces include WiFi or Bluetooth type connections ("Wi-Fi" is a registered trade mark of the Wi-Fi Alliance, and "Bluetooth" is a registered trade mark of Bluetooth SIG, Inc.).

The communications interface can be used to send data to and from the device, for example in downloading data to the computer on the device usage. The communications interface is therefore preferably a two-way interface. This data can then be used in building a user's profile, which can be analysed to recommend, and optionally enable automated delivery of, other fragrances that are likely to be appreciated by the user or to alter the functionality of the device depending on its past usage. Functionality of the device that may be changed could include whether the device emits sound and light when operated. The amount of fragrance emitted may also be controlled, for example to adjust to a consumer preference. In optional embodiments with multiple fragrances in a cartridge, the mix of the fragrances may also be adjusted.

The device optionally comprises an indicator element that is configured to operate according to a characteristic of the device, which has the advantage that different colours or intensities of light or different sounds can be used to indicate the characteristic, which may for example be the fill state of the cartridge or the type of fragrance held in the cartridge. The problem of ensuring that different cartridges can be distinguishable without the need to change tooling can be addressed by configuring the device to cause the light-emitting element to emit a different type of light with a different cartridge. For example, an indicator element in the form of a light-emitting element may emit a different colour light that corresponds with the fragrance in the cartridge. The light-emitting element may also be used in different ways to indicate the fill status of the cartridge or to indicate to the user that the device is in a state that requires attention such as a low battery state or when connected to a computer. In a simple way, the light-emitting element can provide internal illumination of the cartridge that allows a liquid level to be more easily viewed. In a more complex way, a fill level may be known or derived by the base, for example derived from past usage or from a level indicator in the cartridge, and the light emitting element used to indicate to the user when the cartridge is about to run out.

Optionally the base comprises the light-emitting element, although in alternative embodiments the cartridge may comprise the light-emitting element.

The characteristic of the cartridge may be indicated by indicia provided on the cartridge, which the base is configured to read and operate the light-emitting element accordingly. Such indicia may for example be a series of markings on the cartridge that the base can read, for example a barcode. Other types of indicia may include an RFID tag, a coded series of microswitches in the base that are activated when the cartridge is engaged with the base, or a memory unit located in the cartridge and read by the base when the base and cartridge are mechanically engaged with each other.

The base may be configured to operate the light-emitting element according to a quantity of fragrance medium present in the cartridge. For example, the base may operate the light emitting element in a different way when the fragrance is about to run out, such as by causing the light emitting element to flash rather than emit a constant light.

In certain embodiments, the light-emitting element is configured to emit a selectable range of colours and the base is configured to cause the light-emitting element to emit a light of a colour corresponding to the characteristic of the cartridge. The characteristic of the cartridge may correspond to a type of fragrance medium stored in the cartridge. For example, a particular fragrance may be linked with the colour red, while a different fragrance is linked with the colour blue. The device may then be configured to operate the light-emitting element accordingly whenever a cartridge of a particular type is used.

Alternatively, the cartridge may be configured to emit light of a colour corresponding to the characteristic of the cartridge when illuminated by light emitted by the light-emitting element. For example, the cartridge may contain a fluorescent pigment that emits visible light when illuminated by ultraviolet light emitted by the light-emitting element. The fluorescent pigment may be contained in the fragrance medium contained in the cartridge or may be part of the cartridge material itself, for example mixed in with a polymer used to form a part of the cartridge such as a window component.

The fragrance contained in the cartridge may also have an optical characteristic (such as its colour or opacity) that can be used in conjunction with the light-emitting element to cause the cartridge to emit an appropriate light according to the type of cartridge being used. The light emitted may be altered according to the indications received from the server, if the device is also configured according to the second aspect of the invention.

A device according to one or more embodiments of the invention may be configured to emit one or more audible signals in addition to, or instead of, light, where the audible signals correspond to a characteristic of the cartridge being used. For example, an audible signal in the form of a particular tone or series of tones may be emitted by an audio driver provided on the device (preferably in the base) in response to the device being operated. The audio driver may be provided instead of, or in addition to, the above-mentioned light-emitting element, the device being configured to operate the audio driver according to the type of fragrance medium present in the cartridge. The audio driver may be a separate component, or may be provided by the fragrance dispenser itself, for example if the fragrance dispenser comprises a piezoelectric driver that can be used for generating audible sounds by vibrating the fragrance dispensing membrane over a range of different frequencies, as for example disclosed in US 2009/054116.

According to a second aspect of the invention there is provided a method of operating a device according to the first aspect, where the device comprises a communications interface configured to communicate with a computer, the method comprising:
connecting the device to the computer via the communications interface;
transmitting data from the device memory, including data on usage of the device, to the computer;
transmitting the data from the computer to a remote server; and
receiving from the server at the computer one or more indications based on the data transmitted to the server.

The indications may be transmitted from the server to the computer, and optionally transmitted also to the device connected to the computer, for example if the indications relate to operation of the device. The communications interface is therefore preferably a two-way interface, allowing data to be sent to and from the device when connected to the computer.

This method of the invention has the advantage that personalised, rather than averaged, indications are provided to a user of the device, which depend on that particular user's usage of the device, for example how often and at what times the dispenser is operated and which fragrance is used, together with the duration of use.

The indications may be based on the usage data of the device, and may include one or more of: an indication of the status of the cartridge; an indication of the usage history of the dispenser; an indication of a selection of a different type of cartridge; and an indication of a change of configuration of the device, for example to allow the device to emit a difference combination of fragrances (provided multiple sub-cartridges, or a multiple-fragrance cartridge, are present). These types of indications can be used to inform the user when a cartridge needs changing or refreshing, to allow the device to be configured so that its functionality is changed according to how it has previously been used, and to provide a recommendation of a different fragrance based on use of a current or past fragrance. Examples of such recommendations could include recommending a fragrance based on past usage of one or more similar or complementary fragrances, or based on a recommendation derived from a popularity poll conducted on the server among a plurality of users operating such devices.

According to a further aspect of the invention there is provided a method of operating a hand-portable fragrance-dispensing device configured to communicate with a computer via a communications interface, the method comprising the steps of:
connecting the device to the computer via the communications interface;
transmitting over the communications interface from the device to the computer information relating to the device;
transmitting the information from the computer to a remote server; and
receiving marketing information relating to the device from the server at the computer.

The information relating to the device may include a current configuration of the device, for example the type of fragrance the device is configured to emit and/or a log of usage of the device.

The marketing information may include one or more of: a recommendation of a different cartridge or fragrance medium for use with the device; and a recommended configuration for operating the device. The recommended configuration may be a set of parameters or codes to enable the device to control a plurality of sub-cartridges, or a single multi-fragrance cartridge, in the device to emit a combination of fragrances. The recommendation may be based on past usage of the device, for example which fragrances have been used when and for how long.

Other embodiments and advantages of the above aspects of the invention will become apparent from the following detailed description of preferred embodiments.

Aspects and embodiments of the invention are described in further detail below by way of example and with reference to the enclosed drawings in which:
figure 1a is a front perspective view of an exemplary fragrance dispensing device;
figure 1b is a back perspective view of the device of figure 1 a
figure 2 is a perspective view of a cartridge portion of the exemplary device;
figure 3 is a back perspective view of the cartridge portion of the device;
figure 4 is a schematic diagram of the exemplary device connected to a computer that is itself connected to a remote server via the internet;
figures 5a and 5b are front and rear perspective exploded views of an exemplary embodiment of a cartridge comprising multiple fragrance chambers; and
figures 6a and 6b are front and rear perspective views of the multiple fragrance chambers of the cartridge of the cartridge of figures 5a and 5b.

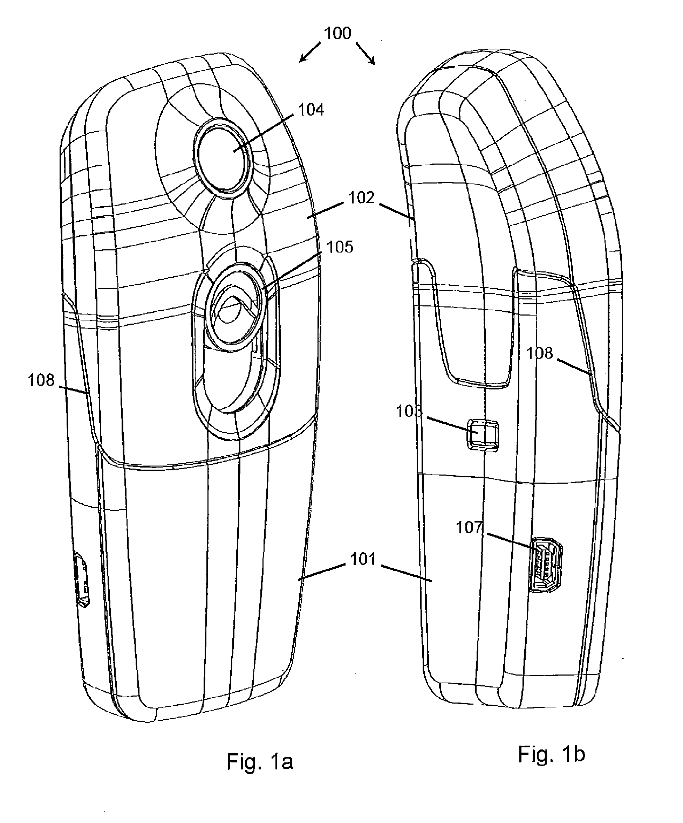

Figures 1 a and 1 b show respective front and rear exterior perspective views of a fragrance dispensing device 100 according to an embodiment of the invention. The device 100 comprises two portions: a base portion 101 and a cartridge portion 102. The base 101 and cartridge 102 are mechanically engaged with each other, and are locked relative to each other with a releasable catch 103. The base and cartridge portions 101, 102 are separable along a join line 108 running around the middle of the device 100. The device 100 is typically of a size that is easily held in one hand and carried in a pocket or handbag, similarly to a handheld portable telephone (or cellphone), and will typically be of the order of 10cm in a longest linear dimension.

The base 101 comprises a battery and electronics that allow for control of the device, while the replaceable cartridge 102 comprises a dispensing actuator located behind a normally-closed cover 104, the dispensing actuator being controlled by an electrical driving signal provided by the electronics within the base 101. The dispensing actuator is controlled by operation of a switch 105 on the cartridge 102. The dispensing actuator may for example be of the type disclosed in FR2929861 or FR2929862, modified accordingly.

The base 101 also comprises a communications interface 107 which, in the embodiment illustrated in figure 1 a and 1 b, is in the form of a USB (Universal Serial Bus) connection. This interface 107 allows the device 100 to communicate with another device such as a personal computer, which enables data stored on the device 100 to be downloaded and communicated to the computer and optionally to a remote server to enable additional functionality of the device such as user interaction according to usage of the device. Various other functions may also be enabled through the use of a communications interface 107, such as allowing a battery in the base 101 of the device 100 to be recharged via the USB connection or to allow the device 100 to be used as a portable storage medium. The device 100 may additionally be configured to be remotely operated by means of the communications interface 107. Other forms of communications interface may alternatively be used, and in some embodiments a wireless interface may be used.

The base 101 may further comprise a light-emitting element, which may comprise one or more light-emitting diodes. In preferred embodiments, the light-emitting element is configured to emit a range of colours of visible light, for example by means of a choice of three different coloured LEDs (typically blue, red and green).

By altering the intensity of the different individual colours, a broad range of apparent colours can be selected, the particular colour preferably being chosen according to a characteristic of the cartridge 102 being used. The light-emitting element may for example be a single tri-colour LED. The light-emitting element may be contained in the base or may, in alternative embodiments, be contained in the cartridge. The light-emitting element may, in certain embodiments, be configured to emit light outside the normal visible spectrum, for example in the form of ultraviolet light that could be used for example to illuminate a fluorescent component in the cartridge and thereby indirectly illuminate the cartridge. The fluorescent component may be a fluorescent pigment that is incorporated in the cartridge or in the fragrance medium contained in the cartridge.

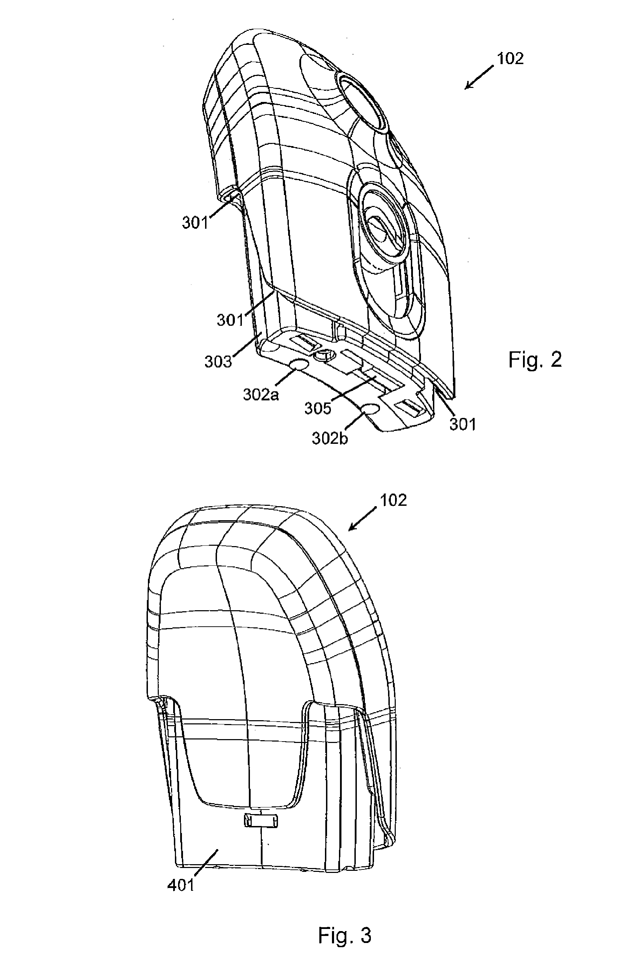

Figure 2 shows a perspective view of the cartridge 102. A light-transmitting (i.e. transparent or translucent) portion 303 of the cartridge 102 is configured to fit against a light-emitting element in the base 101 to allow light from the light-emitting element to be transmitted through the cartridge and illuminate a selected portion, such as a container for a fragrance medium. This is advantageous for the user because the fill state of the cartridge as well as the type of fragrance the cartridge contains can be easily viewed, and is also advantageous in terms of manufacturing the device because the active components are not contained in the replaceable cartridge but in the base unit.

Figure 3 shows a view of the rear of the cartridge 102. In the embodiment shown, the rear cover 401 is at least partly composed of a light-transmitting material, such as a clear or tinted polymeric material. This allows a level of a liquid fragrance medium held within the internal volume 402 to be viewed, and allows the aforementioned light-emitting element to illuminate the cover 401 when lit by the base 101. The light-emitting element is therefore preferably located in the device such that, when operated, the rear cover 401 of the cartridge 102 is illuminated.

The embodiments describe herein indicate a single cartridge and dispensing actuator assembly. In alternative embodiments, the cartridge may comprise multiple sub-cartridges. Each sub-cartridge may be provided with a dispensing assembly, or alternatively each sub-cartridge may be configured to feed a common dispensing assembly. In such alternative embodiments, the quantity of fragrance emitted from each cartridge can be controlled, allowing for a range of fragrance combinations to be emitted from the device. The individual sub-cartridges may be interchangeable as a combined unit or individually.

When the actuation mechanism is operated, a sequence of operations is carried out in the following order. First, a dispensing surface aperture of the dispensing actuator is uncovered. Once the dispensing actuator is completely uncovered, an electrical signal is provided from the base 101 to activate the dispensing device. The light-emitting element may be operated in a particular way during dispensing, optionally in conjunction with the device emitting a preset sound. Optionally, the electronics in the base could be configured to leave the light-emitting element activated for a set time, for example a few seconds. This enables the user to be able to check the fluid level and illumination of the cartridge. Other functions may be enabled at the same time by the actuation mechanism, such as causing the device to emit preset sounds.

Additional functionality may also be included such as operation of the actuation mechanism causing the device to generate a sound, for example by means disclosed in US 2009/0054116. The device may thereby be configured to cause an audible warning, for example relating to the charging state of the battery to indicate when recharging is required or when the level of fragrance medium is low. Additional light emitting elements may be included for similar purposes. The device may be configured to calculate a volume of fragrance medium remaining in the cartridge from knowledge of use of the device, i.e. from a total volume of fragrance that has been emitted. The light-emitting element may be operated differently once the device calculates that the fragrance medium has reached a low level, for example by reducing the brightness of the element or by flashing the element to warn the user that the cartridge needs replacing.

Illumination of the device by means of a light emitting element would also be desirable when the unit is digitally connected, so that the user can tell that a connection has been made. The type of light (i.e. the colour or switching sequence) may vary depending on how the device is being used or what state the device is in, such as a charging state resulting in the light emitting element being caused to flash or emit a characteristic colour indicating charging..

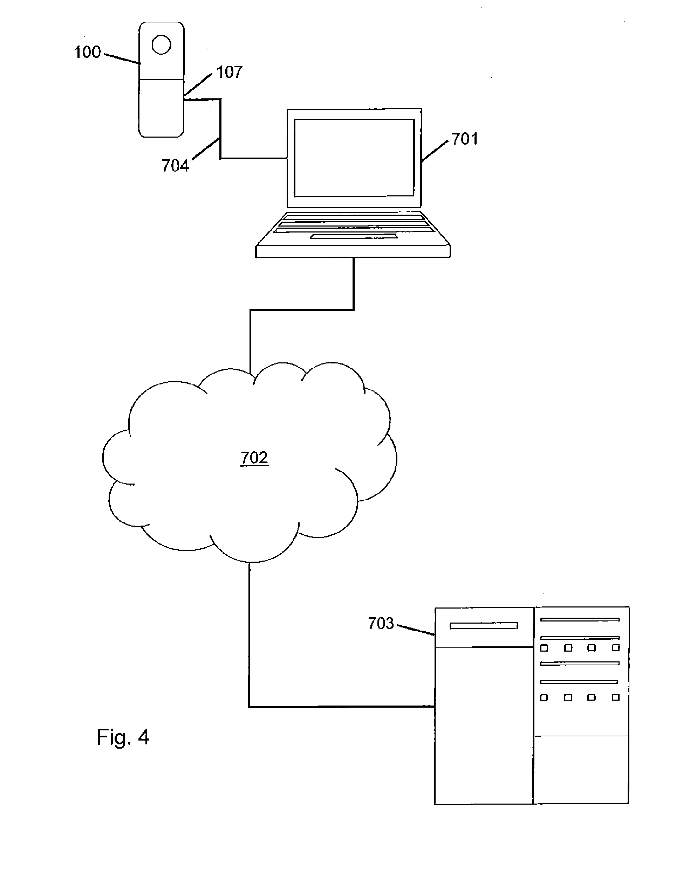

Figure 4 illustrates schematically a device 100 connected to a computer 701 via a link 704 connected to the communications interface 107 on the device 100. With the device 100 connected, information from the device 100 is transmitted to the computer 701, for example including identification codes relating to the base and/or cartridge and data stored on the device relating to usage of the device 100. The computer is further connected to a remote server 703 via the internet 702 and transmits at least a portion of the data received from the device 100 to the remote server 703, which then creates or retrieves a profile associated with that device and allows the computer 701 access to a personalised website. A user of the computer 701 can then access information relating to other cartridges available for the device and obtain recommendations and other marketing information that may be specific or related to the device or the user's profile.

In certain embodiments, as well as emitting light the device may additionally be configured to emit sound and/or vibration when activated.

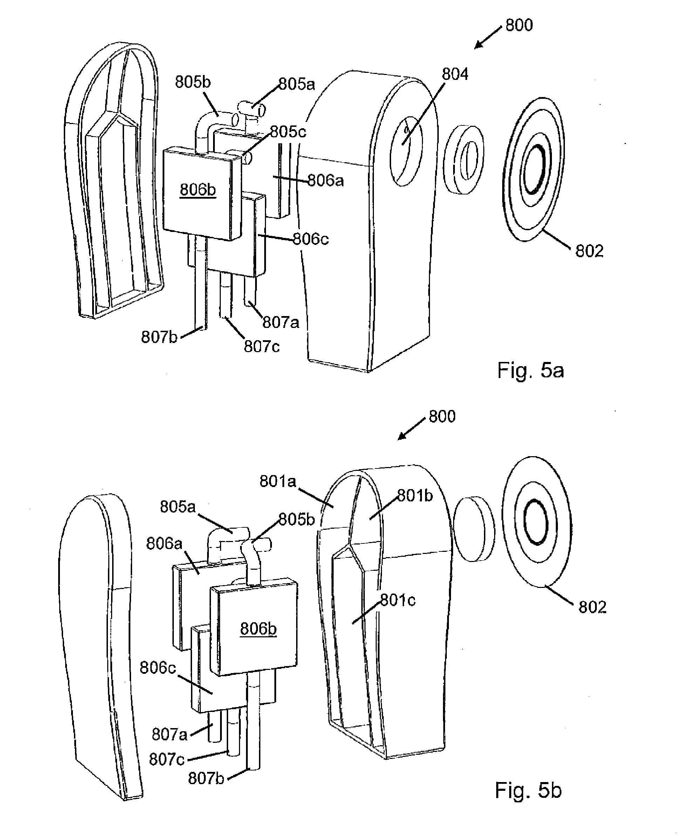

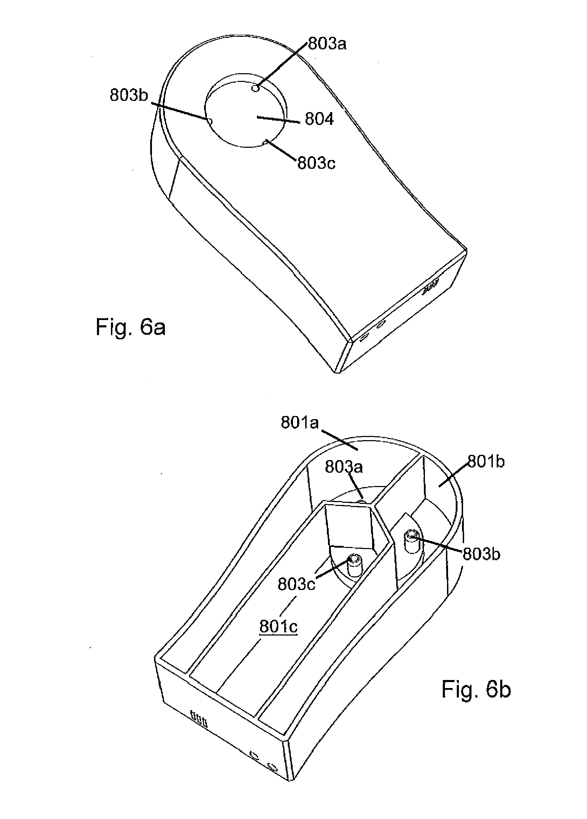

An alternative embodiment of a cartridge for use with a fragrance dispensing device according to the invention is illustrated in figures 5 and 6. The cartridge 800, shown in exploded view in figures 5a and 5b, comprises a plurality of sub-cartridges or chambers 801 a-c, each of which is configured to contain a fragrance medium when the cartridge 800 is assembled. The cartridge 800 is preferably, though not essentially, designed such that the volume of each of the chambers 801 a-c is substantially equal. The fragrances contained within each chamber are preferably different, such that a combination can be used to produce a range of fragrances dispensed by the cartridge 800. The fragrance medium within each chamber 801 a-c is fed to a common dispensing actuator 802. The use of multiple chambers 801 a-c with a common dispensing actuator allows for the cartridge to be used in a number of different ways. If, for example, each of the pumps 806a-c is operated at a constant rate, a uniform mixture of fragrances will be dispensed by the dispensing actuator while there is sufficient fragrance medium in each chamber. This would, however, be effectively no different to having the mixture of fragrances in a single cartridge, although this mode of operation may be advantageous in enabling the dispenser to emit a combination of multiple fragrances that cannot be held together in a single medium for significant periods of time. An advantage of having multiple pumps is in the ability to select which of the fragrances is to be dispensed at any time, and to change the proportion of the fragrances being dispensed. As an example, the base unit may control the pumps according to a particular sequence of operation, which may be based on a user's preferences. The type of fragrance dispensed may, for example, be dependent on the day, time of day or location at which the device is used. Alternatively, the pumps may be controlled to dispense fragrances in sequence so that each chamber is emptied before fragrance from a subsequent chamber is used. Other ways of operating a multi-chamber cartridge may also be envisaged.

The feed ports 803a-c connected to the mixing chamber 804 are connected to feed tubes 805a-c, each of which are connected to respective pumps 806a-c. The pumps 806a-c are configured to draw a fragrance medium contained within the respective chambers 801 a-c through inlet (or dip) tubes 807a-c under control from a base unit connected to the cartridge 800. Electrical signals provided to each pump 806a-c control the rate at which each pump is operated, and therefore how much of each fragrance medium is provided to the mixing chamber 804.

Figures 5 and 6 illustrate simplified views of the cartridge 800, with certain features of mechanical and electrical connections with a corresponding base unit not explicitly shown. It is to be understood, however, that in practice the configuration of the cartridge 800, in terms of the electrical and mechanical connections with the base unit, may be similar to the cartridges described in the embodiments described above.

In alternative configurations, the cartridge 800 may instead be provided with more than one dispensing actuator, for example having one actuator connected to each of the chambers via a separate feed tube and pump. An advantage of such an arrangement would be that of providing more control over the combination of fragrances dispensed, together with being more responsive to changes in the proportion of fragrances supplied by each pump, but at the expense of additional manufacturing costs and complexity for the cartridge as a whole. The use of a single dispensing actuator is therefore generally preferred.

Other embodiments are intentionally within the scope of the invention, which is defined by the appended claims.

## Claims

1. A hand-portable fragrance dispensing device (100) comprising a base (101) and a cartridge (102) configured for mechanical engagement with each other, the cartridge configured to contain a fragrance medium, the device comprising an electrically-driven dispenser configured to dispense an aerosol of the fragrance medium from the cartridge under control from a driver contained in the base,
wherein the device comprises a communications interface (107) configured to communicate with a computer and is configured to transmit an identification code for the device over the communications interface when connected to the computer.

2. The device of claim 1 wherein the device (100) comprises a memory and is configured to log data on usage of the device and to store the data in the memory.

3. The device of claim 2 wherein the base (101) comprises the memory.

4. The device of any one of claims 1 to 3 wherein the communications interface (107) comprises a connection for a wired link to the computer.

5. The device of any preceding claim wherein the communications interface is configured for wireless communication with the computer.

6. The device of any preceding claim wherein the cartridge (102) comprises the electrically-driven dispenser.

7. The device of any preceding claim wherein the device comprises an indicator element, the base being configured to operate the indicator element according to a characteristic of the cartridge.

8. The device (100) of claim 7 wherein the base (101) comprises the indicator element.

9. The device (100) of claim 7 or claim 8 wherein the characteristic of the cartridge (102) is indicated by indicia provided on the cartridge and the base (101) is configured to read the indicia and operate the indicator element accordingly.

10. The device (100) of any one of claims 7 to 9 wherein the base (101) is configured to operate the indicator element according to a quantity of fragrance medium present in the cartridge (101).

11. The device of any one of claims 7 to 10 wherein the indicator element comprises a light-emitting element.

12. The device of any one of claims 7 to 11 wherein the indicator element comprises an audio transducer.

13. The device (100) of claim 11 wherein the light-emitting element is configured to emit a selectable range of colours and the base is configured to cause the light-emitting element to emit a light of a colour corresponding to the characteristic of the cartridge (102).

14. The device (100) of claim 11 or claim 13 wherein the cartridge is configured to emit light of a colour corresponding to the characteristic of the cartridge when illuminated by light emitted by the light-emitting element.

15. The device (100) of any one of claims 7 to 14 wherein the characteristic of the cartridge (102) corresponds to the type of fragrance medium stored in the cartridge.

16. The device of any preceding claim wherein the cartridge (102) comprises a plurality of sub-cartridges.

17. The device of claim 16 wherein each sub-cartridge comprises an electrically-driven dispenser.

18. The device of claim 16 wherein each sub-cartridge is configured to provide a fragrance medium to a common electrically-driven dispenser.

19. A method of operating a device (100) according to any one of claims 1 to 18, the method comprising:
connecting the device to the computer via the communications interface (107);
transmitting identification data from the device to the computer;
transmitting the identification data from the computer to a remote server; and
receiving from the server at the computer one or more indications based on the data transmitted to the server.

20. The method of claim 19 wherein data on usage of the device are transmitted from the device to the computer over the communications interface (107).

21. The method of claim 20 wherein the indications are based on the usage data of the device (100).

22. The method of any one of claims 19 to 21 wherein the indications include one or more of:
an indication of the status of the cartridge (102);
an indication of the usage history of the device (100); and
an indication of a selection of a different type of cartridge (102).
